# EUROPEAN PATENT APPLICATION

(11) **EP 1 554 983 A1**
(43) Date of publication of application: **20.07.2005**
(21) Application number: 04004567.6
(22) Date of filing: 27.02.2004
(51) Int. Cl.: A61B 17/12, A61L 31/16

(54) **Bioabsorbable vasoocclusive coil**

(30) Priority: 16.01.2004 JP 2004009545
(71) Applicant: Medico's Hirata Inc., Osaka-shi, Osaka 530-0003 (JP)
(72) Inventor: Satake, Mitsuo, Chuo-ku Tokyo 104-0045 (JP); Yokogawa, Toru, Medico's Hirata Inc., Nishi-ku Osaka-shi Osaka 550-0002 (JP); Seki, Keiichi, Medico's Hirata Inc., Nishi-ku Osaka-shi Osaka 550-0002 (JP)
(74) Representative: TER MEER STEINMEISTER & PARTNER GbR

(57) **Abstract**

Poly(L-lactic acid) was melt-spun at 200° C and drawn to obtain a hollow monofilament 0.15 mm in outside diameter and 0.12 mm in inside diameter. The monofilament was cut to a length of 8 cm. Each of opposite ends of the cut piece was wound around a stainless steel mandrel, 1 mm in diameter, one-half turn, held in J-shaped curl, heat-treated at 150° C and then cooled. In this way, a bioabsorbable vasoocclusive coil 3 of the invention was prepared which had a J-shaped curved portion 1 at each of opposite ends thereof and a hollow portion 2 over the entire length thereof. The hollow portion can be filled with a drug such as carcinostatic or antitumor agent, permitting the drug to exhibit a sustained release effect. When the follow portion is filled with an X-ray contrast medium, the coil can be delivered to a desired intravascular site reliably, while the process of treatment can be observed more reliably.

## Description

### BACKGROUND OF THE INVENTION

### (a) Field of the Invention

The present invention relates to coils for use in blood vessels of patients for occluding the blood vessel, and more particularly to an occlusive coil made from a material absorbable by the living body. The vasoocclusive coil provides a plug in the blood vessel for use in treating internal hemorrhage or aneurysm or suppressing the flow of blood to a tumor.

### (b) Description of the Prior Art

Vasoocclusive coils are made by winding a wire into a helical form of small diameter. Some vasoocclusive coils are made from a primary coil thus obtained by helically winding a wire, by winding the coil into a helical form of large diameter, i.e., into a secondary coil. The vasoocclusive coil is inserted substantially straight into a catheter, then forced by a pusher to the desired site within a blood vessel, pushed out of the catheter and retained at the site. The publication of JP-A No. 1997-276280 discloses such coils.

Conventional vasoocclusive coils are made of a metal wire such as stainless steel wire, platinum wire or the like. The metal coil produces artifact images in MRI diagnosis, presenting difficulty in conducting diagnosis, and remains in the living body semipermanently without changing in vivo. Accordingly, when the occluded vascular site is to be surgically removed later, the metal coil becomes an obstacle, possibly raising difficulties in surgical excision. Furthermore the coil placed in remains as it is in vivo even after completely serving its function. A surgical operation becomes necessary again for removing the coil retained.

### SUMMARY OF THE INVENTION

An object of the present invention is to provide a bioabsorbable vasoocclusive coil which is free of the forgoing problems encountered with metal coils.

The present invention provides a bioabsorbable vasoocclusive coil to be transported as placed in a catheter to a desired site in a blood vessel and thereafter pushed out of the catheter to the intravascular site, the vasoocclusive coil being formed from a flexible hollow monofilament of a bioabsorbable polymer.

The hollow monofilament forming the coil is preferably 0.015 mm to 0.4 mm, more preferably 0.02 mm to 0. 2 mm in outside diameter and preferably 0.01 mm to 0.3 mm in inside diameter.

### BRIEF DESCRIPTION OF THE DRAWINGS

FIG. 1(a) is a plan view showing a bioabsorbable vasoocclusive coil comprising a hollow monofilament and having opposite ends in a J-shaped curve, and FIG. 1(b) is a view in section taken along the line b-b in FIG. 1(a);
FIG. 2 is a plan view showing a bioabsorbable vasoocclusive coil comprising a hollow monofilament and having opposite ends each in the form of a circular loop;
FIG. 3 is a plan view showing a helically wound bioabsorbable hollow vasoocclusive coil;
FIG. 4 is a plan view showing a bioabsorbable hollow vasoocclusive coil comprising a helically wound coil having opposite ends in a J-shaped curve; and
FIG. 5 is a plan view showing a hollow bioabsorbable vasoocclusive coil comprising a helically wound coil of primary diameter and made by further winding the coil.

### DESCRIPTION OF THE PREFERRED EMBODIMENTS

First, a description will be given of a bioabsorbable polymer serving as the material of the hollow monofilament.

Desirable bioabsorbable polymers are aliphatic polyester bioabsorbable polymers. Examples of such polymers are poly(α-hydroxy acids) such as poly(glycolic acid) and poly (lactic acid), poly-ε-caprolactone, polydioxanone, etc. These aliphatic polyesters are generally 60 to 200° C in melting point, -60 to 100° C in glass transition point and about 100,000 to about 300,000 in weight average molecular weight. Also useful are copolymers comprising glycolic acid as the main component unit, copolymers comprising lactic acid as the main component unit, copolymers comprising ε-caprolactone as the main component unit, and copolymers comprising dioxanone as the main component unit. Such polymers may be used in mixture.

Among the aliphatic polyesters mentioned above, especially preferable are poly(glycolic acid), poly(lactic acid), copolymers comprising glycolic acid as the main component unit and copolymers comprising lactic acid as the main component unit because these polymers are excellent in bioabsorbability and high in safety in vivo and because glycolic acid or lactic acid which results from the decomposition of such polymers is absorbable in vivo.

Poly(lactic acid) is preferred because this polymer is excellent in mechanical strength, has good fiber properties, and is low in cost, transparent and amenable to coloring. With respect to safety in the living body, poly (lactic acid) is not only safe itself but is advantageous in that there is no need to add a plasticizer to this polymer unlike conventional polymer materials. Especially desirable as poly (lactic acid) is poly(L-lactic acid) which comprises L-lactic acid units because this polymer is excellent in mechanical strength and affords desired fiber properties.

The poly (lactic acid) is not limited to homopolymers but may be a lactic acid copolymer comprising other monomer component which is copolymerizable with lactic acid monomer or lactide. Examples of such monomer components are dicarboxylic acids, polyhydric alcohols, hydroxycarboxylic aids, lactones, etc. which have at least two ester linkage forming functional groups.

Poly (lactic acid) can be prepared by a known process as disclosed, for example, in the publication of JP-A No. 1995-3861, publication of JP-A No. 1984-96123, and Proceedings of the Symposium on High Polymers in Japan, Vol. 44, pp. 3198-3199, namely, from lactic acid by direct dehydration condensation, or by the ring-opening polymerization of lactic acid cyclic dimmer lactide. It is also desirable to permit an increased quantity of the monomer to remain in poly (lactic acid) or to add an agent for promoting absorption in vivo to the polymer in order to give poly (lactic acid) enhanced bioabsorbability.

It is desired to incorporate an X-ray contrast medium (barium sulfate, gold powder, iodine contrast medium or the like) into the material for the hollow monofilament, i.e. , into the bioabsorbable polymer. This makes it possible to transport the coil to the desired intravascular site reliably and to observe the process of treatment more reliably.

The shapes of bioabsorbable vasoocclusive coils of the present invention will be described next.

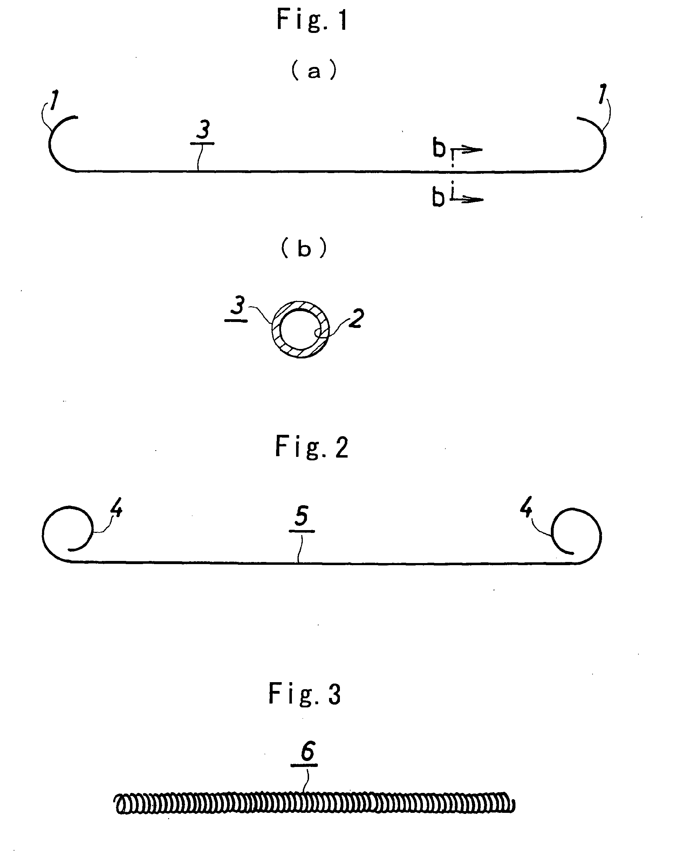

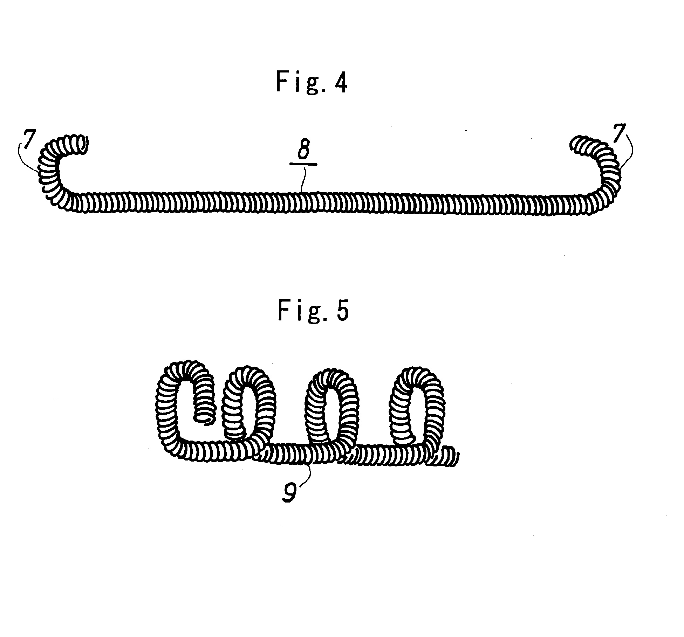

The bioabsorbable vasoocclusive coils of the present invention include, for example, one comprising a hollow monofilament of a bioabsorbable polymer and having opposite ends in a J-shaped curve (see FIG. 1) , one comprising a hollow monofilament of a bioabsorbable polymer and having opposite ends each in the form of a circular loop (see FIG. 2), one obtained by helically winding a hollow monofilament of a bioabsorbable polymer around a straight mandrel and thereafter removing the mandrel from the resulting winding (see FIG. 3), one comprising this helical coil (see FIG. 3) and having opposite ends in a J-shaped curve (see FIG. 4), one comprising a secondary coil obtained by winding a hollow monofilament of a bioabsorbable polymer into a helical form of small diameter to prepare a primary coil and further winding the primary coil into a helical form of large diameter (see FIG. 5), etc.

The hollow monofilament is produced by extruding a resin composition serving as a material from the nozzle of an extruder. The outside diameter and the inside diameter of the hollow monofilament are determined by suitably selecting conditions such as the outside diameter and inside diameter of the nozzle, the extrusion rate and haul-off speed of the resin composition, etc. The opposite ends of the coil can be given the J-shaped curve as shown in FIG. 1 by placing a straight hollow monofilament into a die having a J-shaped groove, heat-treating the die at a temperature close to the melting point, or heat-treating a hollow monofilament as wound around a mandrel by one-half turn at each end thereof, and cooling the resulting monofilament. The vasoocclusive coil thus obtained is pushed, as placed as it is in a catheter, through a blood vessel by a pusher to a desired intravascular site, where the coil is pushed out of the catheter. The coil then restores the J-shaped curved ends although the curvature slightly increases. Before the vasoocclusive coil is inserted into the catheter, the hollow monofilament having opposite ends in a J-shaped curve may be heat-treated at a temperature not lower than the glass transition point of the material polymer but lower than the melting point thereof, thereby straightened at its J-shaped ends and then cooled as it is. The straight vasoocclusive coil is inserted into the catheter, transported to the desired site in the same manner as above and delivered to the site by being pushed out of the catheter. Although the pushed-out coil is straight upon delivery, the opposite ends are restored gradually to the J shape by virtue of the body temperature.

The coil having opposite ends in the form of a circular loop and shown in FIG. 2 can be made also in the same manner as above.

The helical coil shown in FIG, 3 can be obtained by helically winding a hollow monofilament of a bioabsorbable polymer around a straight mandrel, heat-treating the winding at a temperature close to the melting point of the polymer, cooling the winding and thereafter removing the mandrel from the winding. The mandrel may comprise a metal wire. The coil can be placed at a desired site in the same manner as above by inserting the coil as it is into a catheter, transporting the coil to the desired site through a blood vessel and pushing out the coil from the catheter.

The helical coil shown in FIG. 4 comprises a helical coil as shown in FIG. 3 and having opposite ends in a J-shaped curve. This coil is obtained by helically winding a hollow monofilament around a mandrel having J-shaped bent opposite ends, heat-treating the winding at a temperature approximate to the melting point of the polymer, cooling the winding and thereafter removing the mandrel from the winding.

The helical coil shown in FIG. 5 comprises a secondary coil obtained by winding a hollow monofilament of a bioabsorbable polymer into a helical form of small diameter to prepare a primary coil and further winding the primary coil into a helical form of large diameter, and is made by helically winding the hollow monofilament around a mandrel which is bent helically, heat-treating the resulting winding at a temperature approximate to the melting point of the polymer, cooling the winding and thereafter removing the mandrel from the winding. The bent mandrel need not always be helical but can be in a random shape.

To be satisfactory for use, the mandrel is one made of a material having a melting point above the melting point of the bioabsorbable polymer, such as stainless steel, copper or aluminum. The mandrel is preferably 0.03 mm to 2 mm, more preferably 0.04 mm to 1 mm, in diameter.

The helical coil is preferably 0.05 mm to 2 mm, more preferably 0.07 mm to 1 mm, in the diameter of helix. The vasoocclusive coil is preferably 1 cm to 30 cm in length.

The bioabsorbable vasoocclusive coil of the present invention is used by the method to be described next.

The coil of the invention is passed in a substantially straight form through a catheter and transported to a desired site within a blood vessel and pushed out of the catheter to the intravascular site. The coil may have a J-shaped curved end or looped end or may be helical in its entirety. The coil holds shape memory, and when pushed out of the catheter after passing substantially straight through the catheter, the coil restores the shape.

Various known methods are usable for moving the vasoocclusive coil of the invention through the catheter to a desired location and releasing the coil from the catheter outwardly thereof at the location. These methods include, for example, a method wherein a pusher is used for pushing the coil through the catheter to the desired location and is electrically cut off from the coil at the location, a method wherein the coil is connected to the catheter by screw-thread engagement therewith, and a method wherein the coil is locally given an increased thickness so as to be reliably fixedly positionable within the catheter, and is released from the catheter at the desired vascular site.

The vasoocclusive coil of the present invention consists mainly of a bioabsorbable polymer and is gradually decomposed as by hydrolysis to disappear when retained in the living body for a prolonged period of time. The period taken for disappearance varies with the conditions involved in vivo, the shape of coil, etc.

It is desired that the hollow portion of the bioabsorbable vasoocclusive coil be filled with a carcinostatic, antitumor agent or like drug. This attains vasoocclusion and permits the drug to exhibit a sustained release effect. Alternatively, the hollow portion can be filled with an X-ray contrast medium component (barium sulfate, gold powder, iodine contrast medium or the like). Consequently, the coil can be delivered to the desired intravascular site reliably, while the process of treatment can be observed more reliably.

The bioabsorbable vasoocclusive coil of the present invention has the following advantages.

The vasoocclusive coil of the invention is made from a bioabsorbable material, therefore produces no artifact in MRI diagnosis, is usable for the purpose of temporary vasoocclusion and is unlikely to cause trouble unlike metal coils when the occluded vascular site is surgically removed later.

Since the bioabsorbable vasoocclusive coil of the invention is hollow, the hollow portion can be filled with a carsinostatic, antitumor agent or like drug. This attains vasoocclusion while producing a sustained release effect of the drug. When the hollow portion is filled with an X-ray contrast medium component (barium sulfate, gold powder, iodine contrast medium or the like) , the coil can be delivered to the desired intravascular site reliably, while the process of treatment can be observed more reliably.

The present invention will be described below in greater detail with reference to examples, whereas the invention is not limited to these examples.

### [Example 1]

Poly(L-lactic acid) (product of Shimadzu Corp., trade name "Lacty," 200,000 in weight average molecular weight, 175° C in melting point) was melt-spun at 200° C and drawn to obtain a hollow monofilament 0.15 mm in outside diameter and 0.12 mm in inside diameter. The monofilament was cut to a length of 8 cm. Each of opposite ends of the cut piece was wound around a stainless steel mandrel, 1 mm in diameter, one-half turn, held in J-shaped curl, heat-treated at 150° C and then cooled. In this way, a bioabsorbable vasoocclusive coil 3 was prepared which had a J-shaped curved portion 1 at each of opposite ends thereof and a hollow portion 2 over the entire length thereof as shown in FIG. 1.

### [Example 2]

The hollow monofilament of poly (L-lactic acid) produced in Example 1 and having an outside diameter of 0.15 mm and an inside diameter of 0.12 mm was cut to a length of 60 cm. Each of opposite ends of the cut piece was wound around a stainless steel mandrel, 1 mm in diameter, one turn, held in the form of a circular loop, heat-treated at 150° C and then cooled. In this way, a bioabsorbable hollow vasoocclusive coil 5 was prepared which had a circular loop portion 4 at each of opposite ends thereof as shown in FIG. 2.

The vasoocclusive coil was inserted as stretched substantially straight into the base end of a microcatheter measuring 1 mm in outside diameter and 110 cm in entire length and filled with physiological saline, and advanced to the catheter distal end, from which the coil was pushed out of the catheter. The coil thereafter restored itself to the shape of circular loop at each end thereof.

### [Example 3]

The hollow monofilament of poly (L-lactic acid) produced in Example 1 and having an outside diameter of 0.15 mm and an inside diameter of 0.12 mm was cut to a length of 60 cm. The cut piece was helically wound around a straight stainless steel mandrel, 2 mm in diameter, heat-treated at 150° C and then cooled. The winding was thereafter removed from the mandrel. In this way, a bioabsorbable hollow vasoocclusive coil 6 was prepared which was about 5 cm in length and in the form of a helical winding as shown in FIG. 3.

### [Example 4]

In the same manner as in Example 1, a hollow monofilament of poly(L-lactic acid) was produced which had an outside diameter of 0.1 mm and an inside diameter of 0.08 mm. The monofilament was cut to a length of 100 cm. The cut piece of hollow monofilament was helically wound around a straight stainless steel mandrel, having a diameter of 1 mm and opposite ends in a J-shaped curve, heat-treated at 150° C and then cooled. The winding was thereafter removed from the mandrel. In this way, a bioabsorbable hollow vasoocclusive coil 8 was prepared which was in the form of a helical winding having a J-shaped curve portions 7 at each of opposite ends thereof as shown in FIG. 4.

### [Example 5]

In the same manner as in Example 1, a hollow monofilament of poly(L-lactic acid) was produced which had an outside diameter of 0.1 mm and an inside diameter of 0.08 mm. The monofilament was cut to a length of 200 cm. The cut piece of hollow monofilament was helically wound around a mandrel prepared by winding a straight stainless steel wire having a diameter of 1 mm into a substantially helical form with a diameter of 4 mm, heat-treated at 150° C and then cooled. The winding was thereafter removed from the mandrel. In this way, a bioabsorbable hollow vasoocclusive coil 9 was prepared which was in the form of a winding having a secondary diameter of 4 mm and made by winding a helical winding having a primary diameter of 1 mm as shown in FIG. 5.

### [Example 6]

A substance having high consistency and containing a carcinostatic was filled into the hollow potion of the bioabsorbable hollow vasoocclusive coil prepared in Example 3 and having the shape shown in FIG. 3.

## Claims

1. A bioabsorbable vasoocclusive coil to be transported as placed in a catheter to a desired site in a blood vessel and thereafter pushed out of the catheter to the intravascular site, the vasoocclusive coil being formed from a flexible hollow monofilament of a bioabsorbable polymer.

2. A bioabsorbable vasoocclusive coil according to claim 1 which holds shape memory and restores the shape thereof after being pushed out of the catheter.

3. A bioabsorbable vasoocclusive coil according to claim 1 wherein the monofilament is 0.015 mm to 0.4 mm in outside diameter.

4. A bioabsorbable vasoocclusive coil according to claim 1 wherein the monofilament is 0.01 mm to 0.3 mm in inside diameter.

5. A bioabsorbable vasoocclusive coil according to claim 1 wherein the bioabsorbable polymer is an aliphatic polyester bioabsorbable polymer.

6. A bioabsorbable vasoocclusive coil according to claim 1 wherein the bioabsorbable polymer is a poly(lactic acid).

7. A bioabsorbable vasoocclusive coil according to claim 1 which is formed by winding the hollow monofilament of the bioabsorbable polymer helically around a mandrel.

8. A bioabsorbable vasoocclusive coil according to claim 7 wherein the mandrel has a bent portion at at least one end thereof.

9. A bioabsorbable vasoocclusive coil according to claim 7 wherein the mandrel is in the form of a straight rod or has a helical or random form.

10. A bioabsorbable vasoocclusive coil according to claim 1 which comprises a secondary coil formed by winding the hollow monofilament of the bioabsorbable polymer into a helical form of small diameter and further winding the primary coil into a helical form of large diameter.
